(19) Europäisches Patentamt
European Patent Office
Office européen des brevets

(11) Publication number: 0 399 814
A2

(12) # EUROPEAN PATENT APPLICATION

(21) Application number: 90305633.1

(22) Date of filing: 23.05.90

(51) Int. Cl.5: C07C 235/34, C07C 235/36, C07C 235/38, C07C 235/40, A61K 31/165, C07D 235/30, C07D 285/135, C07D 215/38, C07D 409/12, C07D 317/60, C07D 333/68, //C07D277/46, C07D213/75

(30) Priority: 23.05.89 JP 129344/89
07.08.89 JP 204222/89

(43) Date of publication of application:
28.11.90 Bulletin 90/48

(84) Designated Contracting States:
AT BE CH DE DK ES FR GB GR IT LI LU NL SE

(71) Applicant: Sankyo Company Limited
5-1 Nihonbashi Honcho 3-chome Chuo-ku
Tokyo(JP)

(72) Inventor: Naruto, Shunji, c/o Sankyo Company Limited
No. 2-58, 1-chome, Hiromachi
Shinagawa-ku, Tokyo 140(JP)
Inventor: Matsuda, Keiichi, c/o Sankyo Company Limited
No. 2-58, 1-chome, Hiromachi
Shinagawa-ku, Tokyo 140(JP)
Inventor: Sugano, Yuichi, c/o Sankyo Company Limited
No. 2-58, 1-chome, Hiromachi
Shinagawa-ku, Tokyo 140(JP)
Inventor: Sugimoto, Masahiko, c/o Sankyo Company Limited
No. 2-58, 1-chome, Hiromachi
Shinagawa-ku, Tokyo 140(JP)

(74) Representative: Gibson, Christian John Robert et al
MARKS & CLERK 57/60 Lincoln's Inn Fields
London WC2A 3LS(GB)

(54) **Phenol derivatives for promoting human nerve growth factor.**

(57) Phenol derivatives of use in promoting formation of nerve growth factor are of the general formula (Ia) or (Ib)



Xerox Copy Centre

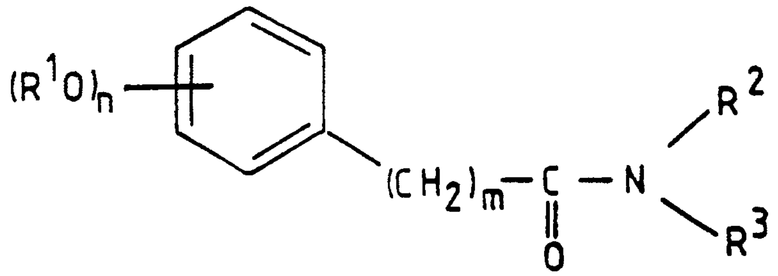

(Ia)

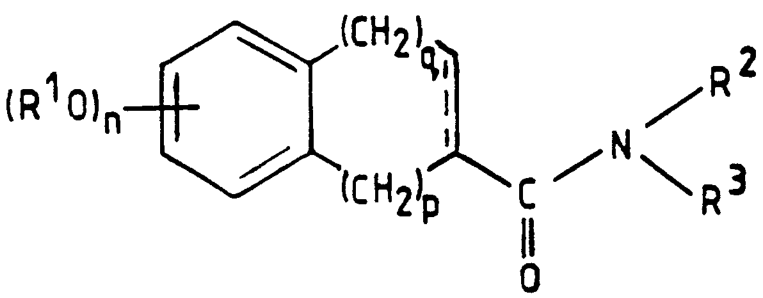

(Ib)

wherein,
each $R^1$ is hydrogen or a hydroxy-protecting group; either
$R^2$ is alkyl, cycloalkyl, cycloalkyl condensed with aryl, aryl, aralkyl or heterocyclic; and
$R^3$ is hydrogen or a group $R^2$;
or
$R^2$ and $R^3$, with the nitrogen atom to which they are attached, represent a cyclic amino group;
m is 1 to 6;
n is 1 to 3, provided that when n is 2 or 3 then two adjacent groups $R^1$ may jointly form a hydroxy-protecting group;
p is 0 or 1 to 3;
q is 0 or 1 to 3; and
the bond marked with a dashed line may be a single bond or a double bond); or a pharmaceutically acceptable salt thereof.

## PHENOL DERIVATIVES FOR PROMOTING HUMAN NERVE GROWTH FACTOR

The present invention relates to novel compounds which promote the production and secretion of human nerve growth factor.

The discovery of nerve growth factor, "NGF", was reported by Levi-Montaleini et al. in 1954. NGF is one of the nutritive and growth factors necessary to maintain the growth and function of the nerve system. It has been recognized recently that NGF can accelerate the repair of peripheral nerve damage and the repair of damage to the functioning of the central nervous system, particularly in Alzheimer's disease and brain ischemia. However, administration of NGF itself is not desirable, because it is a high molecular protein, with a molecular weight of 13,000 for the monomeric form and 26,000 for the dimeric form, and more generally because of concerns for safety.

It is known that neurotransmitters based on catechols, such as adrenaline or noradrenaline, and compounds analogous to catechol, can promote NGF production.

An object of the present invention is the development of drugs effective as promoters of human nerve growth factor. A particular object is the provision of such drugs with reduced side effects, particularly nerve excitation activity. Further objects of this invention include the provision of pharmaceutical compositions for use in treatment of peripheral nerve damage and treatment of damage to the functioning of the central nervous system, especially in Alzheimer's disease and brain ischemia.

In accordance with the present invention, there are provided phenol derivatives of the general formula (Ia) and (Ib)

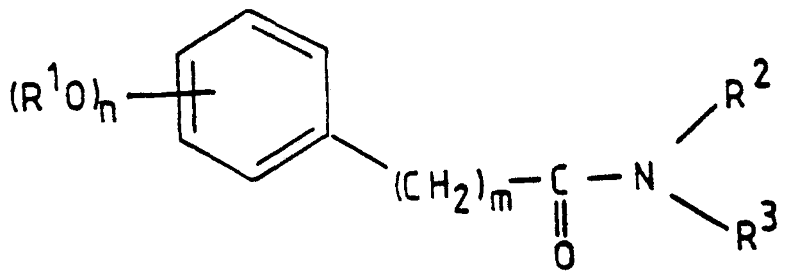

(Ia)

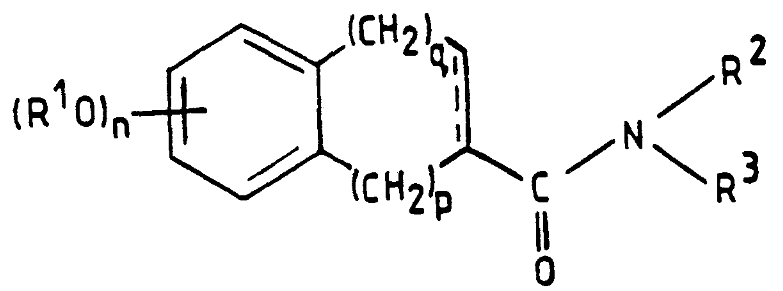

(Ib)

(wherein,
each $R^1$ is the same or different and represents a hydrogen atom or a hydroxy-protecting group; either
$R^2$ represents an alkyl group, a cycloalkyl group, a cycloalkyl group condensed with an aryl group, an aryl group, an aralkyl group or a heterocyclic group; and
$R^3$ represents a hydrogen atom or a group $R^2$;
or
$R^2$ and $R^3$, together with the nitrogen atom to which they are attached, represent a cyclic amino group;
m represents an integer of from 1 to 6;
n represents an integer of from 1 to 3, provided that when n is 2 or 3 then two adjacent groups $R^1$ may together form a joint hydroxy-protecting group;
p represents 0 or an integer of from 1 to 3;
q represents 0 or an integer of from 1 to 3; and

the bond marked with a dashed line may be a single bond or a double bond)
and pharmacologically acceptable salts thereof.

Examples of the preferred compounds in accordance with the present invention comprise those wherein $R^1$ represents a hydrogen atom; a hydroxy-protecting group effective as a protecting group in a chemical reaction and thus corresponding to a compound of formula (Ia) or (Ib) which is a synthetic intermediate; or a hydroxy-protecting group hydrolyzable in vivo upon administration and thus corresponding to a compound of formula (Ia) or (Ib) which is a pro-drug.

Examples of hydroxy-protecting groups for $R^1$ effective as a protecting group in a chemical reaction include:

an alkyl group having 1 to 6 carbon atoms, such as a methyl, ethyl, propyl, isopropyl, butyl, isobutyl, sec-butyl, tert-butyl, pentyl or hexyl group;

an aliphatic acyl group, for example,

an alkylcarbonyl group of which the alkyl group has 1 to 20 carbon atoms, such as a formyl, acetyl, propionyl, butyryl, isobutyryl, pentanoyl, pivaloyl, valeryl, isovaleryl, hexanoyl, octanoyl, lauroyl, palmitoyl or stearoyl group,

a haloalkylcarbonyl group of which the alkyl group has 1 to 6 carbon atoms and which typically has 1 to 3 chlorine or bromine atoms, suoh as a chloroacetyl, dichloroacetyl, trichloroacetyl or trifluoroacetyl group,

an alkoxyalkylcarbonyl group of which the alkoxy and the alkyl group each has 1 to 6 carbon atoms, such as a methoxyacetyl group,

or an unsaturated alkylcarbonyl group of which the alkyl group has 1 to 6 carbon atoms, such as a (E)-2-methyl-2-butenoyl group;

an aromatic acyl group, for example,

an arylcarbonyl group such as a benzoyl, α-naphthoyl or β-naphthoyl group,

a haloarylcarbonyl group which typically has 1 to 3 chlorine or bromine atoms, such as a 2-bromobenzoyl or 4-chlorobenzoyl group,

an alkylarylcarbonyl group of which the alkyl group has 1 to 6 carbon atoms, such as a 2,4,6-trimethylbenzoyl or 4-toluoyl group,

an alkoxyarylcarbonyl group of which the alkoxy group has 1 to 6 carbon atoms, such as a 4-anisoyl group,

a nitroarylcarbonyl group such as a 4-nitrobenzoyl or 2-nitrobenzoyl group,

an alkoxycarbonylarylcarbonyl group, of which the alkoxy group has 1 to 6 carbon atoms, such as a 2-(methoxycarbonyl)benzoyl group, or

an arylarylcarbonyl group such as 4-phenylbenzoyl group;

a tetrahydropyranyl or tetrahydrothiopyranyl group, which may be substituted, such as a tetrahydropyran-2-yl, 3-bromotetrahydropyran-2-yl, 4-methoxytetrahydropyran-4-yl, tetrahydrothiopyran-2-yl or 4-methoxytetrahydrothiopyran-4-yl group;

a tetrahydrofuranyl or tetrahydrothiofuranyl group, which may be substituted, such as a tetrahydrofuran-2-yl or tetrahydrothiofuran-2-yl group;

a silyl group, for example,

a trialkylsilyl group of which the alkyl group has 1 to 6 carbon atoms, such as a trimethylsilyl, triethylsilyl, isopropyldimethylsilyl, tert-butyldimethylsilyl, methyldiisopropylsilyl, methyldi-tert-butylsilyl or triisopropylsilyl group, or

a mono- or di-alkyl, di- or mono-aryl silyl group of which the alkyl group has 1 to 6 carbon atoms and the aryl group has 6 or 10 carbon atoms, being a silyl group substituted with 1 or 2 alkyl groups and correspondingly 2 or 1 aryl groups, such as a diphenylmethylsilyl, diphenylbutylsilyl, diphenylisopropylsilyl or phenyldiisopropylsilyl group;

an alkoxymethyl group, for example,

an alkoxymethyl group of which the alkoxy group has 1 to 6 carbon atoms, suoh as a methoxymethyl, 1,1-dimethyl-1-methoxymethyl, ethoxymethyl, propoxymethyl, isopropoxymethyl, butoxymethyl or t-butoxymethyl group,

an alkoxyalkoxymethyl group of which each alkoxy group has 1 to 6 carbon atoms, such as a 2-methoxyethoxymethyl group, or

a haloalkoxymethyl group of which the alkoxy group has 1 to 6 carbon atoms and which typically has 1 to 3 chlorine or bromine atoms, such as a 2,2,2-trichloroethoxymethyl or bis(2-chloroethoxy)methyl group;

a substituted ethyl group, for example, an alkoxyethyl group of which the alkoxy group has 1 to 6 carbon atoms, such as a 1-ethoxyethyl, 1-methyl-1-methoxyethyl or 1-(isopropoxy)ethyl group, or

a haloethyl group which typically has 1 to 3 chlorine or bromine atoms, such as a 2,2,2-trichloroethyl group;

an aralkyl group, for example, an alkyl group substituted with 1 to 3 aryl groups of which the alkyl group has 1 to 3 carbon atoms and each aryl group may optionally be substituted with 1 to 3 substituents which

are the same or different and each is a $C_{1-6}$ alkyl, $C_{1-6}$ alkoxy, nitro, halogen or cyano group, or two such substituents jointly form a substituent group which may be an alkylenedioxy group having 1 to 4 carbon atoms, such as a benzyl, phenethyl, 3-phenylpropyl, α-naphthylmethyl, β-naphthylmethyl, diphenylmethyl, triphenylmethyl, α-naphthyldiphenylmethyl, 9-anthrylmethyl, 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl, 4-cyanobenzyl, 4-cyanobenzyldiphenylmethyl, bis(2-nitrophenyl)methyl, or a piperonyl group;

an alkoxycarbonyl group, for example, an alkoxycarbonyl group of which the alkoxy group has 1 to 6 carbon atoms, such as a methoxycarbonyl, ethoxycarbonyl, t-butoxycarbonyl or isobutoxycarbonyl group, or an alkoxycarbonyl group of which the alkoxy group has 1 to 6 carbon atoms and which is substituted with a halogen or tri($C_{1-6}$ alkyl)silyl group such as a 2,2,2-trichloroethoxycarbonyl or 2-trimethylsilylethoxycarbonyl group;

an alkenyloxycarbonyl group of which the alkenyloxy group has 1 to 6 carbon atoms, such as a vinyloxycarbonyl or allyloxycarbonyl group; or

an aralkyloxycarbonyl group, the aryl group of which may optionally be substituted with 1 to 2 lower alkoxy or nitro groups and the alkyl group of which has 1 to 3 carbon atoms, such as a benzyloxycarbonyl, 4-methoxybenzyloxycarbonyl, 3,4-dimethoxybenzyloxycarbonyl, 2-nitrobenzyloxycarbonyl or 4-nitrobenzyloxycarbonyl group.

Examples of hydroxy-protecting groups for $R^1$ effective as a protecting group readily hydrolyzable in vivo include:

the said aliphatic acyl group;

the said aromatic acyl group;

an aliphatic acyloxyalkoxycarbonyl group of which the acyl group has 1 to 6 carbon atoms and the alkoxy group has 1 to 6 carbon atoms, such as a pivaloyloxymethoxycarbonyl group;

an aliphatic acyl group substituted with a carboxy group which may optionally be esterified, typically with an alkyl group having 1 to 6 carbon atoms, such as a 3-carboxypropanoyl, 3-ethoxycarbonylpropanoyl or 4-carboxybutanoyl group;

a carbamoyl group which may optionally be substituted with one or more alkyl groups having 1 to 6 carbon atoms, such as a carbamoyl, dimethylcarbamoyl or diethylcarbamoyl group; or

an aminoacid acyl group which may optionally be acylated such as a glycyl, N-acetylglycyl, alanyl, N-acetylalanyl, arginyl or lysyl group.

When n is 2 or 3, then two adjacent groups $R^1$ may together form a joint hydroxy-protecting group, which may be a hydroxy-protecting group effective as a protecting group in a chemical reaction or a hydroxy-protecting group hydrolyzable in vivo.

Examples of such joint hydroxy-protecting groups effective as a protecting group in a chemical reaction include an alkylene group containing 1 to 6 carbon atoms, such as a methylene, methylmethylene, ethylene, propylene, trimethylene, tetramethylene, 1-methyltrimethylene, 2-methyltrimethylene, 3-methyltrimethylene, pentamethylene or hexamethylene group; an alkylidene group containing 1 to 6 carbon atoms, such as a methylidene, ethylidene or isopropylidene group; an aralkylidene group of which the alkyl group has 1 to 3 carbon atoms, such as a benzylidene group; or an alkoxyethylidene group of which the alkoxy group has 1 to 6 carbon atoms, such as a methoxyethylidene or ethoxyethylidene group.

Examples of such joint hydroxy-protecting groups effective as a protecting group hydrolyzable in vivo include the said alkylidene group; an aralkoxyalkylidene group of which the aryl group has 6 or 10 carbon atoms, the alkoxy group has 1 to 3 carbon atoms, and the alkylidene group has 1 to 6 carbon atoms; or the said aralkylidene group.

$R^2$ represents, for example, a linear or branched alkyl group having 1 to 18 carbon atoms such as a methyl, ethyl, propyl, isopropyl, butyl, isobutyl, sec-butyl, tert-butyl, pentyl, hexyl, octyl, dodecyl or octadecyl group; a cycloalkyl group having 3 to 10 carbon atoms which may optionally be condensed with an aryl group, suoh as a cyclopropyl, cyclobutyl, cyclohexyl, norbornyl, adamantyl or 2-indanyl group; an aryl group having 6 or 10 carbon atoms, such as a phenyl or naphthyl group; an aralkyl group containing 7 to 9 carbon atoms such as a benzyl, phenethyl or 3-phenylpropyl group; or a 5- to 7-membered heterocyolic group containing 1 to 3 nitrogen, oxygen or sulphur atoms and which may optionally be condensed with one or more alicyclic, aryl or heterocyclic rings, such as a furyl, thienyl, benzo[b]thienyl, tetrahydrobenzo[b]thienyl, pyrrolyl, pyrazolyl, oxazolyl, isoxazolyl, imidazolyl, thiazolyl, isothiazolyl, triazolyl, tetrazolyl, thiadiazolyl, pyridyl, pyrimidinyl, pyridazinyl, pyrazinyl, indolyl, quinolyl, isoquinolyl, benzimidazolyl or purinyl group.

The ring present in $R^2$ when it is an aryl group, aralkyl group or heterocyclic group may optionally be substituted. For example, the ring may be substituted with 1 to 4 substituents selected from the following

possibilities: an alkyl group having 1 to 6 carbon atoms, such as a methyl, ethyl, propyl, isopropyl, butyl, isobutyl, or hexyl group; an alkoxy group having 1 to 6 carbon atoms, such as a methoxy, ethoxy, propoxy or isopropoxy group, hydroxy group, a halogen atom such as a fluorine, chlorine, bromine or iodine atom, a haloalkyl group having 1 to 6 carbon atoms and typically having 1 to 3 chlorine or bromine atoms, such as a trifluoromethyl or 2,2,2-trifluoroethyl group, an alkylenedioxy group substituted at adjacent positions of the ring, such as a methylenedioxy, ethylenedioxy or propylenedioxy group, a nitro group, an amino group, a di($C_{1-6}$ alkyl)amino group, such as a dimethylamino or diethylamino group, a carboxy group, an alkoxycarbonyl group of which the alkoxy group has 1 to 6 carbon atoms, such as a methoxycarbonyl, ethoxycarbonyl, propoxycarbonyl or isopropoxycarbonyl group, a carbamoyl group, an aliphalic acyl group having 1 to 6 carbon atoms, such as an acetyl, propionyl or butyryl group, or an aromatic acyl group such as a benzoyl, p-chlorobenzoyl, m-chlorobenzoyl or p-anisoyl group.

$R^2$ and $R^3$, together with the nitrogen atom to which they are attached, may optionally form a cyclic amino group, optionally containing a further ring nitrogen, sulphur or oxygen atom and optionally substituted with ring substituents of the kind mentioned for the ring of $R^2$, for example, a 5- or 6-membered cyclic amino group, such as a 1-pyrrolidinyl, piperidino, morpholino, thiomorpholino, 1-piperazinyl or 4-methyl-1-piperazinyl group.

Where $R^1$ and/or $R^2$ contains an asymmetric carbon atom, the compound of the invention can exist in the form of stereoisomers. The present invention embraces individual isomers and any mixture thereof, including a racemic mixture.

The compounds of this invention may exist as pharmacologically acceptable non-toxic salts. Examples of such salts include: preferably, where an acidic group exists in the compound, a salt with an alkaline metal or an alkaline earth metal such as a sodium, potassium or calcium salt; where a basic group exists in the compound, a salt of a hydrohalide such as a hydrofluoride, hydrochloride, hydrobromide or hydroiodide, a salt of an inorganic acid suoh as a nitrate, perchlorate, sulphate or phosphate; a salt of an alkylsulphonic acid such as a methanesulphonate, trifluoromethanesulphonate or ethanesulphonate, a salt of an arylsulphonic acid such as benzenesulphonate or p-toluenesulphonate, a salt of an organic acid such as a fumarate, succinate, citrate, tartarate, oxalate, maleate, etc., or a salt of amino acid such as a glutamate or aspartate.

Examples of preferred compounds of this invention include those wherein n is 2, and especially those which are 2,3-di-($R^1$O) compounds. Further preferred compounds include those which are 3,4-di-($R^1$O) compounds wherein $R^1$ is hydrogen or acetyl, $R^2$ is halophenyl or heterocyclic, and $R^3$ is hydrogen. Another class of preferred compounds comprises those comopunds wherein m is 4 or more. More generally, the preferred compounds of this invention are compounds not disclosed in EP 333522, published on 20 September 1989, being after the Japanese priority dates claimed for the present application.

Preferred compounds of formula (I) also comprise those in which:

$R^1$ represents a hydrogen atom; or a hydroxy-protecting group for a pro-drug, such as an acetyl, benzoyl, pivaloyloxymethyloxycarbonyl, 3-carboxypropanoyl or glycyl group;

$R^2$ represents a cycloalkyl group, such as an adamantyl group; an aryl group such as a phenyl group substituted with 2 or 3 chlorine or bromine atoms; or a heterocyclic group such as a thienyl or tetrahydrobenzo[b]thienyl group substituted with a methoxycarbonyl group;

$R^3$ represents a hydrogen atom;

m is 2, 3 or 4;

n is 2 or 3;

p is 0 or 1; and

q is 1, 2 or 3.

Individual preferred compounds of this invention comprise:

(a) 3-(3,4-diacetoxyphenyl)-N-(2,4,5-trichlorophenyl)propionamide
(b) 3-(3,4-diacetoxyphenyl)-N-(2,3-dichlorophenyl)propionamide
(c) 3-(3-4-diacetoxyphenyl)-N-(2-4-dichlorophenyl)butyramide
(d) N-(2,4-dichlorophenyl)-3-(4-hydroxy-3-dimethylcarbamoyloxyphenyl)propionamide
(e) N-(2,4-dichlorophenyl)-3-(3,4-di-[3-ethoxycarbonylpropanoyloxy]phenyl)propionamide
(f) N-(2,4-dichlorophenyl)-3-(3-carboxypropanoyloxy-4-hydroxyphenyl)propionamide
(g) N-(2,4-dichlorophenyl)-3-(3,4-diglycyloxyphenyl)propionamide
(h) N-(1-adamantyl)-3-(2,3-diacetoxyphenyl)propionamide
(i) N-(2,4-dichlorophenyl)-3-(3,4-dihydroxyphenyl)propionamide
(j) N-(2,4-dichlorophenyl)-6,7-diacetoxy-1,2,3,4-tetrahydro-2-naphthamide
(k) N-(3-quinolyl)-6,7-diacetoxy-1,2,3,4-tetrahydro-2-naphthamide
(l) N-(1-adamantyl)-6,7-diacetoxy-1,2,3,4-tetrahydro-2-naphthamide

(m) N-(2,4-dichlorophenyl)-6,7-diacetoxy-3,4-dihydro-2-naphthamide
(o) N-(3-quinolyl)-6,7-diacetoxy-3,4-dihydro-2-naphthamide
(p) N-(1-adamantyl)-6,7-diacetoxy-3,4-dihydro-2-naphthamide
(g) N-(1-adamantyl)-3-(3,4-diglycyloxyphenyl)propionamide
(r) N-(1-adamantyl)-4-(3,4-diglycyloxyphenyl)butyramide
(s) N-(1-adamantyl)-6,7-diglycyloxy-3,4-dihydro-2-naphthamide
(t) N-(1-adamantyl)-6,7-diglycyloxy-1, 2, 3, 4-tetrahydro-2-naphthamide
(u) N-(2,4-dichlorophenyl)-6,7-diglycyloxy-3,4-dihydro-2-naphthamide
(v) N-(2,4-dichlorophenyl)-6,7-diglyoyloxy-1,2,3,4-tetrahydro-2-naphthamide and pharmaceutically acceptable salts thereof where such salts may be formed (for example, acid addition salts of the glycyl-containing compounds), especially hydrochloride salts.

In general, the compounds of the invention exhibit activity in promoting NGF production and secretion. Moreover, they are low in toxicity. Accordingly, the compounds of the invention may be used for the treatment of dementia, brain ischemia damage and more generally for various kinds of nerve damage.

The present invention provides pharmaceutical compositions which comprise one or more compounds of this invention, together with a pharmaceutically acceptable carrier.

Examples of suitable administration methods include the oral route in the form of a tablet, capsule, granule, powder or syrup, as well as the parenteral route by injection or suppository. The pharmaceutical preparations can be produced according to the conventional manner using the adjuvants generally known in the art, such as one or more of an excipient, binder, disintegrator, lubricant, stabilizer, corrective and the like. The dosage may vary depending upon the symptom, age and other patient factors, but usually is from 1 to 1000 mg per day for an adult. The dosage may be administered once or separately several times a day.

The present invention also provides a process for preparing the compounds of this invention from compounds which are known or may be prepared by conventional techniques. The process involves reacting an acid halide of formula (IIa) or (IIb);

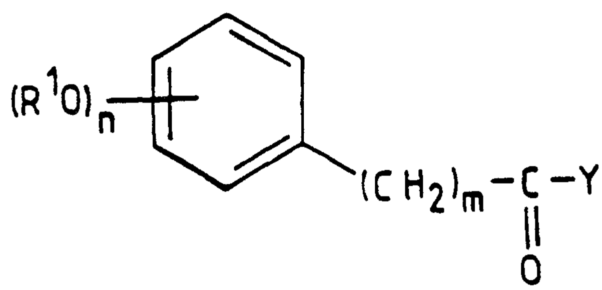

(IIa)

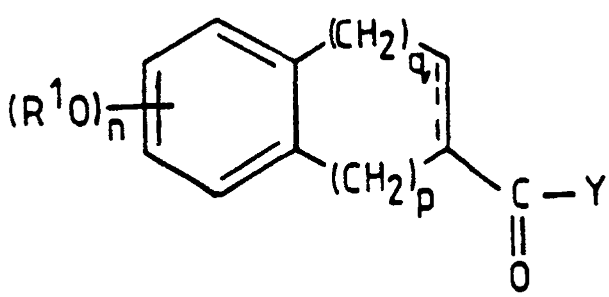

(IIb)

with an amine of formula (III);

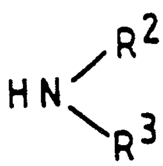

(III)

(where $R^{1'}$ is a hydroxy-protecting group, Y is a halogen atom, and R', $R^2$, $R^3$, m, n, p and q are as defined) and optionally removing the hydroxy-protecting group represented by $R^{1'}$.

The reaction of the acid halide with the amine is suitably carried out in an inert solvent such as a chlorinated hydrocarbon, ether or hydrocarbon, for example, dichloromethane, tetrahydrofuran or toluene, in the presence of a base, typically an organic base such as pyridine, triethylamine, or 4-dimethylaminopyridine. The reaction is usually carried out at a temperature from -10°C to 50°C, preferably 0°C to 30°C. The time required for the reaction varies depending upon the reaction temperature and the nature of the starting material and solvent, but the reaction is normally complete within from 1 to 3 hours.

After completion of the reaction, the desired compound can be recovered from the reaction mixture by conventional means, for example, by adding a water immiscible organic solvent and by distilling off the solvent after washing with water. The choice of solvent used in the recovery is not restricted, provided that it can dissolve the desired compound to some degree. Examples of preferred solvents to be used include aromatic hydrocarbons such as benzene, toluene or xylene; halogenated hydrocarbons such as dichloromethane or chloroform; esters such as ethyl acetate or propyl acetate; ethers such as diethyl ether, tetrahydrofuran, dioxane or dimethoxyethane; alcohols such as methanol, ethanol, n-propanol, isopropanol, n-butanol, isobutanol or isoamyl alcohol; amides such as dimethylformamide, dimethylacetamide or hexamethylphosphorotriamide; and sulphoxides such as dimethylsulphoxide. If desired, the compound can be purified by conventional means, for example by recrystallization, reprecipitation and or chromatography. Furthermore, the recovery can be effected with formation of a salt, which may be adopted for use as such, or converted back to the free compound.

Where the process gives a protected compound which is a synthetic intermediate, the procedure for deprotection varies depending upon the nature of the protecting group $R^1$ and can be carried out by using a method known from the art. Examples of some deprotection procedures are given.

Where the hydroxy-protecting group is a trialkylsilyl group, it can be removed by treatment with a compound which generates a fluoride anion such as tetrabutylammonium fluoride. The solvent used in the reaction is not restricted, provided that it has no adverse effect on the reaction. Examples of preferred solvents include ethers such as tetrahydrofuran and dioxane. The reaction temperature and the time required for the reaction are not critical, but the reaction is normally complete at room temperature within from 10 minutes to 18 hours.

Where the hydroxy-protecting group is an aralkyloxycarbonyl or aralkyl group, it can be removed by contact with a reductant. For example, deprotection can be carried out at room temperature by catalytic reduction in the presence of a catalyst such as palladium on charcoal, platinum or Raney nickel. The reaction is carried out in the presence of a solvent. The identity of the solvent used for the reaction is not limited, provided that it has no adverse effect on the reaction. Examples of preferred solvents include: alcohols such as methanol and ethanol; ethers such as tetrahydrofuran or dioxane; aliphatic acids such as acetic acid; or a mixture of such an organic solvent and water. The reaction temperature and the time required for the reaction vary depending upon the nature of the starting compounds and reductants used, but the reaction is normally complete at a temperature from 0°C to room temperature within from 5 minutes to 12 hours. Alternatively, such a hydroxy-protecting group is removed by reacting metallic lithium or sodium with liquid ammonia or alcohols such as methanol or ethanol at a temperature from -78°C to -20°C.

Where the hydroxy-protecting group is an aliphatic acyl, aromatic acyl or alkoxycarbonyl group, it can be removed by treatment with a base in the presence of a solvent. The nature of the base is not limited, provided that it has no adverse effect on the other parts of the compound. Examples of the preferred base include: metal alcoholates such as sodium methoxide; ammonia solution; alkaline metal carbonates such as sodium carbonate or potassium carbonate; alkaline metal hydroxides such as sodium hydroxide or potassium hydroxide; and a mixture of concentrated ammonia solution and methanol. The solvent used for the reaction is not limited. Examples of preferred solvents include: water; an organic solvent, including alcohols such as methanol, ethanol or n-propanol, or ethers such as tetrahydrofuran or dioxane; or a mixture of water and an organic solvent. The reaction temperature and the time required for the reaction vary depending upon the nature of the starting materials and the base but there is no particular limitation. In order to prevent side reactions, the reaction is normally carried out at a temperature from 0°C to 150°C for a time of from 1 to 10 hours.

Where the hydroxy-protecting group is an alkoxymethyl, tetrahydropyranyl, tetrahydrofuranyl, or substituted ethyl group, it can usually be removed by treatment with an acid in the presence of a solvent. Examples of the preferred acids include: hydrochloric acid, acetic acid-sulphuric acid, p-toluenesulphonic acid or acetic acid. The solvent used for the reaction is not limited, provided that it has no adverse effect for the reaction. Examples of the preferred solvents include: alcohols such as methanol or ethanol; ethers such

as tetrahydrofuran or dioxane; or a mixture of such an organic solvent and water. The reaction temperature and the time required for the reaction vary depending upon the nature of the starting materials and the nature of the acid, but the reaction is typically carried out at a temperature from 0° C to 50° C for a range of from 10 minutes to 18 hours.

Where the hydroxy-protecting group is an alkenyloxycarbonyl group, it can be removed by treatment with a base under similar depropection conditions to those where the protecting group is an aliphatic acyl, aromatic acyl or alkoxycarbonyl group. Still more, where the protective group is an allyloxycarbonyl group, a deprotection procedure using a combination of palladium and triphenylphosphine or nickel tetracarbonyl is convenient and side reactions are minimized.

The following Examples illustrate the preparation of compounds of the invention from known starting compounds or from starting compounds which may be prepared using procedures analogous to those employed for known compounds. Where an $R_f$ is quoted, it is obtained by thin layer chromatography on silica gel Merck Art 5715, thickness 0.25mm, developed with a 1:1 mixture of hexane and ethyl acetate. Reference Examples are included for the preparation of some starting compounds. Examples of NGF promoter activity are included. Formulation Examples are also given.

Reference Example 1

6,7-dimethoxy-2-methoxyoarbonyl-1-tetralone

2. 78 g (0.0135 mol) of 6,7-dimethoxy-1-tetralone was dissolved in 100 ml of dimethyl carbonate and 0.26 g of sodium methoxide was added under a stream of nitrogen. The mixture was refluxed for 5 hours. After cooling, the reaction mixture was neutralized with 2 N acetic acid followed by dilution with ethyl acetate. The mixture was washed successively with an aqueous solution of sodium hydrogen carbonate and 0.5 N hydrochloric acid. The organic layer was dried over anhydrous sodium sulphate and concentrated by distillation. The residue was purified by column chromatography through silica gel to afford 2.8 g (0.0106 mol, 74.5%) of the title compound as white crystals.

Reference Example 2

methyl 6,7-dimethoxy-1,2,3,4-tetrahydro-2-naphthoate

One gram (0.038 mol) of the compound prepared in Reference Example 1 was dissolved in a mixture of 50 ml of acetic acid and 30 ml of methanol, and hydrogenated in the presence of 10% palladium on charcoal as catalyst. After about 170 ml of hydrogen was absorbed, the catalyst was removed by filtration and the filtrate was concentrated by distillation to afford 0.91 g (3. 64 mmol, 96.3%) of the title compound as white crystals.

Reference Example 3

6,7-dihydroxy-1,2,3,4-tetrahydro-2-naphthoic acid

A solution of 0.9 g of the compound prepared in Reference Example 2 dissolved in 30 ml of 47% hydrobromic acid was refluxed for 4 hours. After concentration, the residue was mixed with water under ice cooling and stirred to precipitate crystals which were collected by filtration to afford 0.7 g (3.36 mmol, 98.8%) of the title compound as pale yellow crystals.

Reference Example 4

6,7-diacetoxy-1,2,3,4-tetrahydro-2-naphthoic acid

To a solution of 0.7 g of the compound prepared in Reference Example 3 dissolved in 30 ml of acetic anhydride was added one drop of sulphuric acid and the mixture was stirred for 2 hours. To it was added 30 ml of water followed by stirring for 2 hours. The reaction mixture was diluted with ether and washed successively with water and an aqueous saturated sodium chloride solution. After drying the solution over anhydrous magnesium sulphate, the solvent was stripped off by distillation. The residue was mixed with toluene and concentrated by azeotropic distillation to afford 0.96 g (3.28 mmol, 97.8%) of the title compound.

Reference Example 5

methyl 1-hydroxy-6,7-dimethoxy-1,2,3,4-tetrahydro-2-naphthoate

To a solution of 1.9 g (7.19 mmol) of the compound prepared in Reference Example 1 dissolved in 30 ml of methanol was added 4 g of sodium borohydride at 45° C during 20 minutes. After stirring for 40 minutes, the mixture was concentrated to about one half of original volume and diluted with ethyl acetate. The mixture was washed successively with an aqueous solution of sodium hydrogen carbonate, 0.5 N hydrochloric acid and an aqueous saturated sodium chloride solution. After drying the solution over anhydrous sodium sulphate, the solvent was distilled off to afford 1.9 g (7.14 mmol, 99.3%) of the title compound.

Reference Example 6

methyl 6,7-dimethoxy-3,4-dihydro-2-naphthoate

To a solution of 1.9 g of the compound prepared in Reference Example 5 dissolved in 70 ml of dichloromethane were successively added 2.98 ml of triethylamine and 0.78 ml of thionyl chloride at 0° C, and the mixture was allowed to rise to room temperature followed by stirring for an hour. Then, water was added at 0° C to the reaction mixture. After dilution with water and ethyl acetate, the mixture was washed with an aqueous solution of sodium hydrogen carbonate, 0.5 N hydrochloric acid and an aqueous saturated sodium chloride solution. The organic layer was dried over anhydrous sodium sulphate and the solvent was removed by distillation to afford the title compound.

Reference Example 7

6,7-hydroxy-3,4-dihydro-2-naphthoic acid

To a solution of the compound prepared in Reference Example 6 dissolved in 5 ml of dichloromethane was added dropwise 30 ml of 1 M dichloromethane solution of borontribromide at -78° C under a nitrogen atmosphere. The reaction mixture was allowed to rise to room temperature followed by stirring for 3 hours. After cooling, it was poured into ice water and stirred for an hour followed by extraction with ethyl acetate. The combined extract was washed with an aqueous saturated sodium chloride solution and dried over anhydrous sodium sulphate. After treatment of the solution with active charcoal, the solvent was removed by distillation. The residue was purified by column chromatography through silica gel to afford 0.63 g (3.06 mmol, 42.9%) of the title compound as white crystals.

Reference Example 8

6,7-diacetoxy-3,4-dihydro-2-naphthoic acid

To a solution of 0.7 g of the compound prepared in Reference Example 7 dissolved in 30 ml of acetic

anhydride was added one drop of sulphuric acid, and the mixture was stirred for 2 hours followed by addition of 30 ml of water. After stirring for 2 hours, the reaction mixture was diluted with ether and washed successively with water and an aqueous saturated sodium chloride solution. After drying the solution over anhydrous magnesium sulphate, the solvent was removed by distillation. The residue was mixed with toluene and remaining acetic acid was removed by azeotropic distillation to afford 0.96 g (3.28 mmol, 97.8%) of the tile compound.

Example 1

N-(2,5-dichlorophenyl)-3,4-diacetoxyphenylpropionamide

2,5-Dichloroaniline (1.62 g, 10 mM) and pyridine (1 ml) were dissolved in 20 ml of dichloromethane. To the mixture was added 3,4-diacetoxyphenylpropionyl chloride (2.62 g) with ice cooling under stirring, followed by stirring for 15 minutes at the same temperature. After stirring at room temperature for 30 minutes, the reaction mixture was diluted with water and the organic layer was separated. The aqueous layer was extracted with dichloromethane (2 x 20 ml). The combined extracts were washed with an aqueous solution of sodium hydrogen carbonate and then dilute hydrochloric acid. After drying the washed extract over anhydrous sodium sulphate, the solvent was evaporated to afford 3.2 g of the title compound, m.p. 96 - 97° C

Examples 2 to 47

In a similar manner to the procedure of Example 1, the following tabulated compounds of formula (Ic) were synthesized.

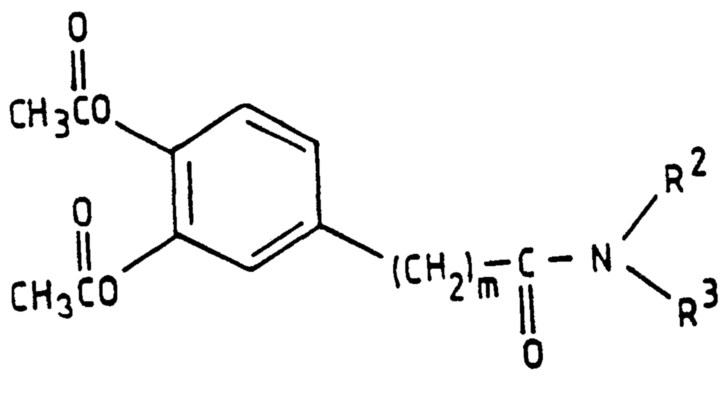

(Ic)

| Example | m | $R^2$ | $R^3$ | mp (°C) |
|---|---|---|---|---|
| 2 | 2 | 2-indanyl | H | oil, $R_f$ 0.38 |
| 3 | 2 | 4-acetylphenyl | H | 101-102 |
| 4 | 2 | 4-methoxyphenyl | H | oil, $R_f$ 0.30 |
| 5 | 2 | 2-benzimidazolyl | H | 127-128 |
| 6 | 2 | 2-(1,3-thiazolyl) | H | 140-141 |
| 7 | 2 | 2-pyridyl | H | 114-116 |
| 8 | 2 | 2-(1,3,4-thiadiazolyl) | H | 175-177 |
| 9 | 2 | 3-pyridyl | H | 147-148 |
| 10 | 2 | 2-(4-methyl-1,3-thiazolyl) | H | 145-147 |
| 11 | 2 | 4-benzoylphenyl | H | 113-114 |
| 12 | 2 | 2-(2-chlorobenzoyl)-4-chlorophenyl | H | oil, $R_f$ 0.55 |
| 13 | 2 | 2,4,5-trichlorophenyl | H | 120-121 |
| 14 | 2 | 3,5-dichlorophenyl | H | 129-131 |
| 15 | 2 | 3,4,5-trichlorophenyl | H | 112-114 |
| 16 | 2 | 3,4-dichlorophenyl | H | 69-73 |
| 17 | 2 | 2,4-dichlorophenyl | H | 139-140 |
| 18 | 2 | 2,3-dichlorophenyl | H | 113-114 |
| 19 | 2 | 2-chlorophenyl | H | 95-96 |
| 20 | 2 | endo-norbornyl | H | 57-60 |
| 21 | 2 | exo-norbornyl | H | 102-105 |
| 22 | 2 | 2-adamantyl | H | 115-116 |
| 23 | 2 | 2-(3-methoxycarbonyl-4,5-dimethylthienyl) | H | 90-95 |
| 24 | 2 | 2-(3,5-diethoxycarbonyl-4-methylthienyl | H | 100-101 |
| 25 | 2 | 1-adamantyl | H | 105-106 |
| 26 | 2 | 6-quinolyl | H | 53-54 |
| 27 | 2 | 2,5-dimethoxycarbonyl-phenyl | H | 123-124 |
| 28 | 2 | 2-amino-4-chlorophenyl | H | 101-103 |
| 29 | 2 | 2,5-difluorophenyl | H | 70-71 |
| 30 | 2 | 2,5-dibromophenyl | H | 98-100 |
| 31 | 2 | 3-quinolyl | H | 107-109 |
| 32 | 2 | 4-chlorophenyl | H | 107-108 |

| | | | | |
|---|---|---|---|---|
| 33 | 2 | 3-chlorophenyl | H | 90-92 |
| 34 | 2 | 2,4,6-trichlorophenyl | H | 158-159 |
| 35 | 2 | phenyl | phenyl | 100-101 |
| 36 | 1 | 2,4-dichlorophenyl | H | 119-120 |
| 37 | 1 | 2-adamantyl | H | 153-154 |
| 38 | 1 | 2-(3-methoxycarbonyl-tetrahydrobenzo[b]thienyl) | H | 112-113 |
| 39 | 2 | 2-(3,4-dihydroxyphenyl)-ethyl | H | glass, $R_f$ 0.31 |
| 40 | 2 | 2,3-dichlorotolyl | H | 106-107 |
| 41 | 2 | 4-hexylphenyl | H | 78-79 |
| 42 | 2 | heptadecyl | H | 79-80 |
| 43 | 1 | 2-(3-methoxycarbonyl-4,5-dimethylthienyl) | H | 179-181 |
| 44 | 3 | 3-quinolyl | H | 122-123 |
| 45 | 3 | 2,4-dichlorophenyl | H | 102-103 |
| 46 | 3 | 1-adamantyl | H | glass, $R_f$ 0.53 |
| 47 | 3 | 2-(3-methoxycarbonyl-tetrahydrobenzo[b]-thienyl) | H | glass, $R_f$ 0.53 |

Examples 48 to 66

In a similar manner to the procedure of Example 1, the following tabulated compounds of formula (Id) were synthesized. In Examples 54 and 56, the substituents $(R^1O)_n$ comprise a 4-hydroxy group and the indicated group at the 3-position. For each of the other Examples, the respective substituents $R^1O$ are the same.

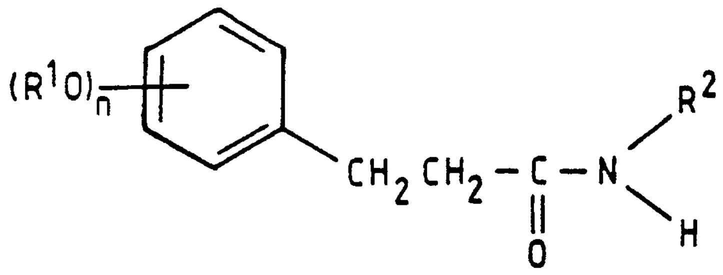

(Id)

| Example | $(R^1O)_n$ | $R^2$ | mp (°C) |
|---|---|---|---|
| 48 | 3,4-methylenoxy | 2-(3-methoxycarbonyl-tetrahydrobenzo[b]-thienyl) | 95-96 |
| 49 | 3,4-methylenoxy | 2-indanyl | 120-121 |
| 50 | 3,4-methylenoxy | 4-acetylphenyl | 147-148 |
| 51 | 3,4-dibenzoyloxy | 2,4-dichlorophenyl | 154-155 |
| 52 | 3,4-dipropanoyloxy | 2,4-dichlorophenyl | 113-114 |
| 53 | 3,4-dihexanoyloxy | 2,4-dichlorophenyl | 102-103 |
| 54 | 3-dimethylcarbamoyloxy, 4-hydroxy | 2,4-dichlorophenyl | 69-70 |
| 55 | 3,4-di-(3-ethoxycarbonyl-propanoyloxy) | 2,4-dichlorophenyl | 89-90 |
| 56 | 3-(3-carboxypropanoyloxy), 4-hydroxy | 2,4-dichlorophenyl | 199-200 |
| 57 | 2,3-diacetoxy | 2,4-dichlorophenyl | 128-129 |
| 58 | 2,3-diacetoxy | 2-(3-methoxycarbonyl-tetrahydrobenzo[b]-thienyl) | 137-138 |
| 59 | 2,3-diacetoxy | 1-adamantyl | 122-123 |
| 60 | 3,5-diacetoxy | 1-adamantyl | oil, $R_f$ 0.47 |
| 61 | 3,5-diacetoxy | 2-(3-methoxycarbonyl-tetrahydrobenzo[b]-thienyl) | 65-67 |

| | | | |
|---|---|---|---|
| 62 | 3,5-diacetoxy | 2,4-dichlorophenyl | 150-151 |
| 63 | 3,4,5-triacetoxy | 2,4-dichlorphenyl | 139-140 |
| 64 | 3,4,5-triacetoxy | 2-(3-methoxycarbonyl-tetrahydrobenzo[b]-thienyl) | 72-73 |
| 65 | 3,4,5-triacetoxy | 1-adamantyl | 137-138 |

Example 66

N-(2,5-dichlorophenyl)-3,4-dihydroxyphenylpropionamide

The amide (1.0 g) prepared in Example 1 was suspended in 20 ml of methanol, and 10 mg of metallic sodium was added to the suspension followed by stirring for 2 hours till the suspension became transparent. The reaction mixture was diluted with 200 ml of water and acidified with dilute hydrochloric acid. The resulting pale yellow precipitate was collected by filtration and recrystallized from ethanol to afford 0.5 g of the title compound, m.p. 130 - 131 °C

Examples 67 to 83

In a similar manner to the procedure of Example 1, the following tabulated compounds of formula (Ie) were synthesized.

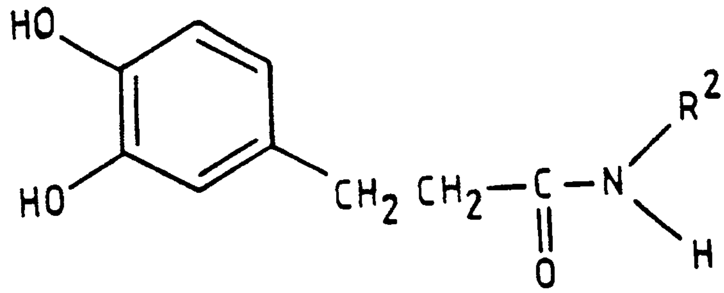

(Ie)

| Example | $R^2$ | $R^3$ | mp (°C) |
|---|---|---|---|
| 67 | 2-(3-methoxycarbonyl-tetrahydrobenzo[b]-thienyl) | H | 168-170 |
| 68 | 2-indanyl | H | 162-163 |
| 69 | 4-acetylphenyl | H | 148-149 |
| 70 | 4-methoxyphenyl | H | glass, $R_f$ 0.60 |
| 71 | 2-thiazolyl | H | 192-195 |
| 72 | 2-pyridyl | H | 193-195 |
| 73 | 2-(1,3,4-thiadiazolyl) | H | 195-197 |
| 74 | 3-pyridyl | H | 243-244 |
| 75 | 2-(4-methyl-1,3-thiazolyl) | H | 172-174 |
| 76 | 4-benzoylphenyl | H | >300 |
| 77 | 2-(2-chlorobenzoyl)-4-chlorophenyl | H | 118-120 |
| 78 | 2,4,5-trichlorophenyl | H | 165-166 |
| 79 | 3,5-dichlorophenyl | H | 140-141 |
| 80 | 3,4,5-trichlorophenyl | H | 149-151 |
| 81 | 3,4-dichlorophenyl | H | 151-153 |
| 82 | 2,4-dichlorophenyl | H | 138-139 |
| 83 | 1-adamantyl | H | 211-212 |

Examples 84 to 86

In a similar manner to the procedure of Example 1, the following tabulated compounds of formula (Ic) were synthesized.

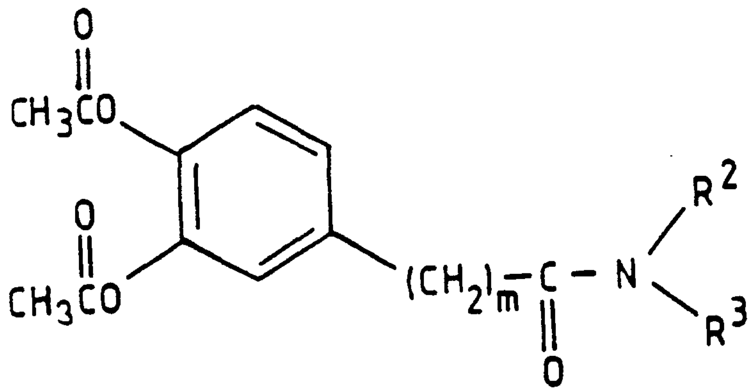

(Ic)

| Example | m | $R^2$ | $R^3$ | mp (°C) |
|---|---|---|---|---|
| 84 | 4 | 2,4-dichlorophenyl | H | 116-117 |
| 85 | 4 | 1-adamantyl | H | oil, $R_f$ 0.26 |
| 86 | 4 | 2-(3-methoxycarbonyl-tetrahydrobenzo[b]-thienyl) | H | oil, $R_f$ 0.66 |

Example 87

A mixture of 1.63 g (6 mM) of the hydroxy compound of Example 66, 1.75 g (10 mM) of tert-butoxyglycine and 2.06 g (10 mM) of DCC in 30 ml of tetrahydrofuran was stirred at room temperature for 3 hours. At the end of this time, the solvent was removed by evaporation under reduced pressure. The residue was subjected to silica gel chromatography and eluted with a 1:1 by volume mixture of hexane and ethyl acetate, to give the tert-butoxyglycine ester of the hydroxy compound. The entire product was dissolved in 10 ml of dioxane. 20 ml of 4N dioxane/HCl was added with ice-cooling to the solution, which was then allowed to stand with ice cooling for 1 hour. The solvent was removed by evaporation under reduced pressure and the residue was thoroughly washed, in turn with diethyl ether and with tetrahydrofuran, to give 2.1 g of the title compound melting above 220 °C.

Examples 88 to 90

In a similar manner to the procedure of Example 87, the following tabulated compounds of formula (If) were synthesized.

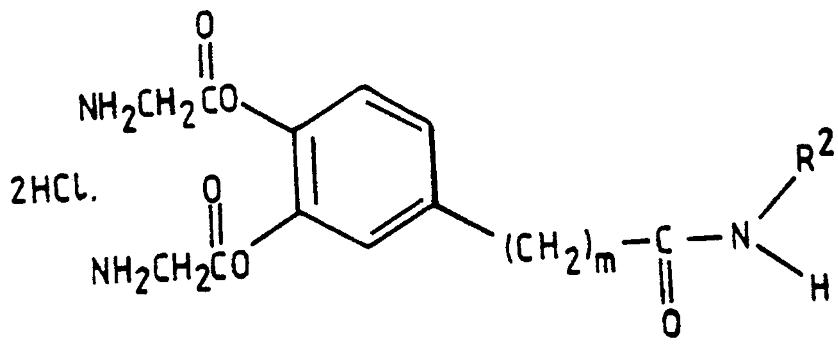

(If)

| Example | m | $R^2$ | mp (°C) |
|---|---|---|---|
| 88 | 3 | 2,4-dichlorophenyl | 196-197 |
| 89 | 2 | 1-adamantyl | 162-164 |
| 90 | 3 | 1-adamantyl | 198 |

Example 91

N-(2,4-dichlorophenyl)-6,7-diacetoxy-1,2,3,4-tetrahydro-2-naphthamide

To a solution of 0.17 g (0.58 mmol) of the compound prepared in Reference Example 4 dissolved in 6 ml of dichloromethane was added one drop of dimethylformamide and the mixture was cooled to 0 °C. After adding 0.4 g of oxalyl chloride, the temperature of the mixture was allowed to rise to room temperature and the mixture stirred for 2 hours. Then, the solvent was completely removed by distillation to dryness. The residue was dissolved in 6 ml of dichloromethane and to the solution were added 0.113 g (0.7 mmol) of 2,4-dichloroaniline and 0.071 mg (0.7 mmol) of triethylamine. After stirring for 3 hours, the mixture was diluted with ethyl acetate and washed successively with water, an diluted aqueous solution of sodium hydrogen carbonate and an aqueous saturated solution of sodium chloride. The organic phase was dried over anhydrous sodium sulphate and the solvent was stripped off by distillation. The residue was purified by column chromatography through silica gel to afford 0.201 g (0. 461 mmol, 79.2%) of the title compound, m.p. 166 - 168 °C

Example 92

N-(3-quinolyl)-6, 7-diacetoxy-1,2,3,4-tetrahydro-2-naphthamide

To a solution of 0.17 g (0.58 mmol) of the compound prepared in Reference Example 4 dissolved in 6 ml of dichloromethane was added one drop of dimethylformamide and the mixture was cooled to 0 °C. After adding 0.4 g of oxalyl chloride, the temperature of the mixture was allowed to rise to room temperature followed by stirring for 2 hours. The reaction mixture was completely freed from the solvent to dryness. The residue was dissolved in 6 ml of dichloromethane and to the solution were added 0.084 g (0.58 mmol) of 3-aminoquinoline and 0.088 mg (0.82 mmol) of triethylamine. After the mixture was stirred for 3 hours, it was diluted with ethyl acetate and washed successively with water, a dilute aqueous solution of sodium hydrogen carbonate and an aqueous saturated sodium chloride solution. The organic phase was dried over anhydrous sodium sulphate and the solvent was removed by distillation. The residue was purified by column chromatography through silica gel to afford 0.207 g (0.495 mmol, 84.1%) of the title compound,

m.p. 194 - 196° C

Example 93

N-(1-adamantyl)-6,7-diacetoxy-1,2,3,4-tetrahydro-2-naphthamide

To a solution of 0.13 g (0.45 mmol) of the compound prepared in Reference Example 4 dissolved in 6 ml of dichloromethane was added one drop of dimethylformamide and the mixture was cooled to 0° C. After adding 0.4 g of oxalyl chloride, the temperature was allowed to rise to room temperature followed by stirring for 2 hours. The reaction mixture was completely freed from the solvent to dryness. The residue was dissolved in 6 ml of dichloromethane and to the solution were added 0.067 g (0.58 mmol) of 1-adamantylamine and 0.068 mg (0.67 mmol) of triethylamine. After the mixture was stirred for 3 hours, it was diluted with ethyl acetate and washed succesively with water, a dilute aqueous solution of sodium hydrogen carbonate and an aqueous saturated sodium chloride solution. The organic phase was dried over anhydrous sodium sulphate and the solvent was removed by distillation. The residue was purified by column chromatography through silica gel to afford 0.114 g (0.268 mmol, 60.3%) of the title compound, m.p. 234 -235° C

Example 94

N-(2,4-dichlorophenyl)-6,7-diacetoxy-3,4-dihydro-2-naphthamide

To a solution of 0.075 g (0.26 mmol) of the compound prepared in Reference Example 8 dissolved in 5 ml of dichloromethane was added one drop of dimethylformamide and the mixture was cooled to 0° C. After adding 0.3 g of oxalyl chloride, the temperature was allowed to rise to room temperature followed by stirring for 2 hours. The reaction mixture was compeltely freed from the solvent to dryness. The residue was dissolved in 5 ml of dichloromethane and to the solution were added 0.050 g (0. 31 mmol) of 2,4-dichloroaniline and 0.039 mg (0.39 mmol) of triethylamine. After the mixture was stirred for 3 hours, it was diluted with ethyl acetate and washed successively with water, a dilute aqueous solution of sodium hydrogen carbonate and an aqueous saturated sodium chloride solution. The organic phase was dried over anhydrous sodium sulphate and the solvent was removed by distillation. The residue was purified by column chromatography through silica gel to afford 0.100 g (0.23 mmol, 89.4%) of the title compound, m.p. 180 - 182° C

Example 95

N-(3-quinolyl)-6,7-diacetoxy-3,4-dihydro-2-naphthamide

To a solution of 0.082 g (0.28 mmol) of the compound prepared in Reference Example 8 dissolved in 5 ml of dichloromethane was added one drop of dimethylformamide and the mixture was cooled to 0° C. After adding 0.3 g of oxalyl chloride, the temperature was allowed to rise to room temperature followed by stirring for 2 hours. The reaction mixture was completely freed from the solvent to dryness. The residue was dissolved in 5 ml of dichloromethane and to the solution were added 0.049 g (0.34 mmol) of 3-aminoquinoline and 0.043 mg (0.42 mmol) of triethylamine. After the mixture was stirred for 3 hours, it was diluted with ethyl acetate and washed successively with water, a dilute aqueous solution of sodium hydrogen carbonate and an aqueous saturated sodium chloride solution. The organic phase was dried over anhydrous sodium sulphate and the solvent was removed by distillation. The residue was purified by column chromatography through silica gel to afford 0.096 g (0.231 mmol, 81.6%) of the title compound, m.p. 214 - 216° C

Example 96

N-(1-adamantyl)-6,7-diacetoxy-3,4-dihydro-2-naphthamide

To a solution of 0.072 g (0.25 mmol) of the compound prepared in Reference Example 8 dissolved in 5 ml of dichloromethane was added one drop of dimethylformamide and the mixture was cooled to 0 °C. After adding 0.3 g of oxalyl chloride, the temperature was allowed to rise to room temperature followed by stirring for 2 hours. The reaction mixture was completely freed from the solvent to dryness. The residue was dissolved in 5 ml of dichloromethane and to the solution were added 0.045 g (0.30 mmol) of 1-adamantylamine and 0.038 mg (0.37 mmol) of triethylamine. After the mixture was stirred for 3 hours, it was diluted with ethyl acetate and washed successively with water, a dilute aqueous solution of sodium hydrogen carbonate and an aqueous saturated sodium chloride solution. The organic phase was dried over anhydrous sodium sulphate and the solvent was removed by distillation. The residue was purified by column chromatography through silica gel to afford 0.082 g (0.194 mmol, 78.4%) of the title compound, m.p. 224 - 226 °C

Examples of NGF promoter activity

Furukawa et al. reported that L-M cells of a fibroblast cell line from mouse connective tissue can produce and secrete a relatively large amount of NGF, and that catecholamines accelerate such production and secretion of NGF (J. Biol. Chem., 261, 6039-6047, 1986). Following the test method described in this Furukawa paper, but using the compounds of the invention and recognized NGF promoters epinephrine, isoproterenol, L-DOPA, and caffeic acid, NGF production and secretion activities were examined. The test compounds of this invention were employed at 10 γ ml, and the known compounds were employed at 20 γ·ml.

Culture medium 199 containing 0.5% peptone was used for culturing L-M cells (for the culture medium 199, see, for instance, Morgan et al., Proc. Soc. Exp. Biol. Med., 73, 1 (1950) or Morgan et al. J. Natl. Cancer Inst., 16, 557 (1955). About 5 x $10^4$ of L-M cells were placed in each well of a culture plate with 24 wells, and cultured using a $CO_2$ incubator (37 °C, 5% $CO_2$) until confluence. After removing the culture medium, the cultured cells were washed once with 199 culture medium containing 0.5% bovine serum albumin (Fraction V, Sigma). The test compounds were added to a specified concentration to a 199 culture medium containing 0.5% bovine serum albumin, and treated with 0.5 ml of L-M cells. After culturing the L-M cells in a $CO_2$ incubator for 24 hours, the medium was recovered and the NGF level was determined.

NGF was determined using an enzyme immunoassay (Korshing and Thoenen, Proc. Natl. Acad. Sci. USA, 80, 3513-3516, 1983). A solution (each 75 µl) of anti-mouse β-NGF antibody (0.3 µg ml, pH 9.6; Boehringer Mannheim) was pipetted into each well of a polystyrene plate with 96 wells. After allowing the plate to stand at room temperature for an hour, the antibody was removed by washing three times with detergent solution. A solution (50 µl) of standard β-NGF (Wako Pure Chemical Industries Ltd.) or the recovered medium (50 µl) for each of the test compounds was pipetted into each well. After allowing the plate to stand at room temperature for 6 - 8 hours, the standard β-NGF or test solution was removed and each well was washed three times. A solution (50 µl) of β-NGF monoclonal antibody (100 mU ml, pH 7.0; Boehringer Mannheim) labelled with β-galactosidase was pipetted into each well. After allowing the plate to stand at 4 °C for 15 - 18 hours, enzyme labelled antibody was removed and the wells washed three times followed by pipetting a solution (100 µl) of chlorophenol red-β-D-galactopyranoside (1 mg ml, pH 7.3; Boehringer Mannheim) into each well. Colour was allowed to develop (2 - 3 hours at room temperature), and the absorbance was determined at 570 nm. The amount of NGF was calculated from a standard curve. The results are expressed as a relative value (%) which is relative to the amount of NGF produced and secreted by cells treated not with the test compounds. The numerical values (% control) are expressed by the mean value in the 3 wells of the control (without addition of the test compounds).

| Known compound | % Control |
|---|---|
| epinephrine | 140 ± 24 |
| isoproterenol | 168 ± 22 |
| L-DOPA | 117 ± 7 |
| caffeic Acid | 123 ± 14 |

| Example Compound | % Control |
|---|---|
| 1 | 201 |
| 11 | 325 |
| 13 | 511 |
| 14 | 239 |
| 15 | 326 |
| 16 | 254 |
| 18 | 508 |
| 23 | 319 |
| 25 | 417 |
| 30 | 381 |
| 31 | 292 |
| 33 | 283 |
| 40 | 279 |
| 45 | 617 |
| 46 | 939 |
| 47 | 265 |
| 51 | 251 |
| 52 | 309 |
| 53 | 201 |
| 55 | 206 |
| 56 | 216 |
| 58 | 361 |
| 59 | 671 |
| 64 | 201 |

| | |
|---|---|
| 67 | 201 |
| 76 | 404 |
| 78 | 488 |
| 79 | 377 |
| 82 | 571 |
| 84 | 857 |
| 85 | 471 |
| 86 | 225 |
| 88 | 300 |
| 89 | 266 |
| 90 | 467 |
| 91 | 575 |
| 93 | 941 |
| 94 | 459 |
| 95 | 341 |
| 96 | 558 |

It can be seen that the compounds of the present invention exhibit excellent activity in promoting NGF production and secretion.

Formulation Example 1

| Capsules | | |
|---|---|---|
| N-(1-adamantyl)-3,4-diacetoxyphenylbutyrylamide (Compound of Example 46) | | 25 mg |
| lactose | | 153.6 mg |
| corn starch | | 100. mg |
| magnesium stearate | | 1.4 mg |
| | Total | 280. mg |

The powder ingredients for the formulation were mixed, passed through a 60 mesh sieve, and 280 mg portions of the resulting powder were packed into No. 3 gelatin capsules.

Formulation Example 2

Capsules

A procedure similar to Formulation Example 1 was adopted with the following ingredients.

| | |
|---|---|
| N-(1-adamantyl)-6,7-diacetoxy-1,2,3,4-tetrahydro-2-naphthamide (Compound of Example 93) | 25 mg |
| lactose | 153.6 mg |
| corn starch | 100 mg |
| magnesium stearate | 1.4 mg |
| Total | 280. mg |

## Claims

1. A phenol derivative of the general formula (Ia) or (Ib)

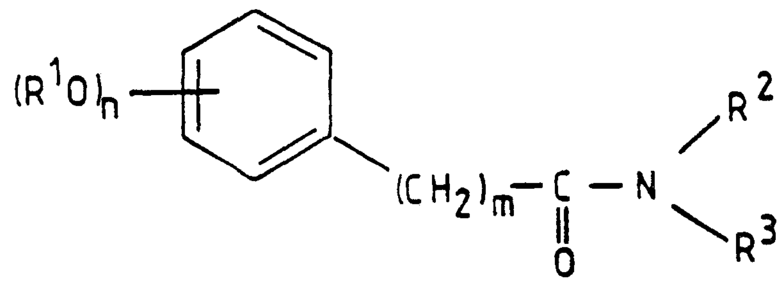

(Ia)

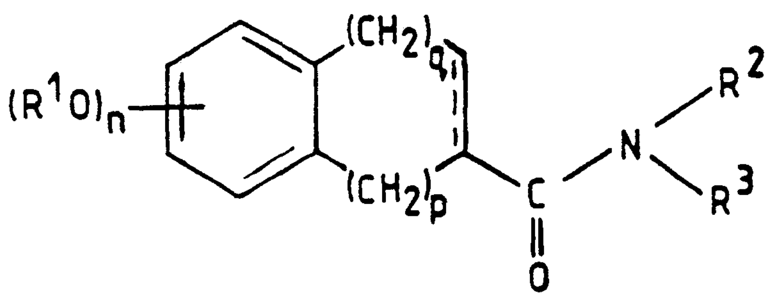

(Ib)

wherein,

each $R^1$ is the same or different and is selected from a hydrogen atom or a hydroxy-protecting group; either $R^2$ is selected from an alkyl group, a cycloalkyl group, a cycloalkyl group condensed with an aryl group, an aryl group, an aralkyl group or a heterocyclic group; and

$R^3$ is selected from a hydrogen atom or a group $R^2$;

or

$R^2$ and $R^3$, together with the nitrogen atom to which they are attached, represent a cyclic amino group;

m represents an integer of from 1 to 6; n represents an integer of from 1 to 3, provided that when n is 2 or 3 then two adjacent groups $R^1$ may together form a joint hydroxy-protecting group;

p represents 0 or an integer of from 1 to 3;

q represents 0 or an integer of from 1 to 3; and the bond marked with a dashed line may be a single bond or a double bond;

and pharmacologically acceptable salts thereof.

2. A compound according to claim 1, wherein $R^1$ is selected from a hydrogen atom or a hydroxy-protecting group for a pro-drug.

3. A compound according to claim 2, wherein $R^1$ is selected from an aliphatic acyl group having 1 to 20 carbon atoms, an arylcarbonyl group, an aliphatic acyloxyalkoxycarbonyl group of which the acyl group has 1 to 6 carbon atoms and the alkoxy group has 1 to 6 carbon atoms, an aliphatic acyl group substituted with

a carboxy group, or an aminoacid acyl group.

4. A compound according to claim 1, 2 or 3, in which $R^2$ is selected from a cycloalkyl group, an aryl group substituted with 2 or 3 chlorine or bromine atoms, or a heterocyclic group substituted with a methoxycarbonyl group.

5. A compound according to any preceding claim, in which $R^3$ represents a hydrogen atom.

6. A compound according to any preceding claim, in which m is 2, 3 or 4.

7. A compound according to any preceding claim, in which n is 2 or 3.

8. A compound according to any preceding claim, in which p is 0 or 1.

9. A compound according to any preceding claim, in which q is 1, 2 or 3.

10. A compound selected from:
3-(3,4-diacetoxyphenyl)-N-(2,4,5-trichlorophenyl)propionamide,
3-(3,4-diacetoxyphenyl)-N-(2,3-dichlorophenyl)propionamide,
3-(3,4-diacetoxyphenyl)-N-(2,4-dichlorophenyl)butyramide,
N-(2,4-dichlorophenyl)-3-(4-hydroxy-3-dimethylcarbamoyloxyphenyl)propionamide,
N-(2,4-dichlorophenyl)-3-(3,4-di-[3-ethoxycarbonylpropanoyloxy]phenyl)propionamide,
N-(2,4-dichlorophenyl)-3-(3-carboxypropanoyloxy-4-hydroxyphenyl)propionamide, N-(2,4-dichlorophenyl)-3-(3,4-diglycyloxyphenyl)propionamide,
N-(1-adamantyl)-3-(2,3-diacetoxyphenyl)propionamide,
N-(2,4-dichlorophenyl)-3-(3,4-dihydroxyphenyl)propionamide,
N-(2,4-dichlorophenyl)-6,7-diacetoxy-1,2,3,4-tetrahydro-2-naphthamide,
N-(3-quinolyl)-6,7-diacetoxy-1,2,3,4-tetrahydro-2-naphthamide,
N-(1-adamantyl)-6,7-diacetoxy-1,2,3,4-tetrahydro-2-naphthamide,
N-(2,4-dichlorophenyl)-6,7-diacetoxy-3,4-dihydro-2-naphthamide,
N-(3-quinolyl)-6,7-diacetoxy-3,4-dihydro-2-naphthamide,
N-(1-adamantyl)-6,7-diacetoxy-3,4-dihydro-2-naphthamide,
N-(1-adamantyl)-3-(3,4-diglycyloxyphenyl)propionamide,
N-(1-adamantyl)-4-(3,4-diglycyloxyphenyl)butyramide,
N-(1-adamantyl)-6,7-diglycyloxy-3,4-dihydro-2-naphthamide,
N-(1-adamantyl)-6,7-diglycyloxy-1,2,3,4-tetrahydro-2-naphthamide,
N-(2,4-dichlorophenyl)-6, 7-diglycyloxy-3,4-dihydro-2-naphthamide, or
N-(2,4-dichlorophenyl)-6,7-diglycyloxy-1,2, 3, 4-tetrahydro-2-naphthamide
and pharmaceutically acceptable salts thereof.

11. A pharmaceutical composition which comprises at least one compound in accordance with any preceding claim, together with a pharmaceutically acceptable carrier.

12. A process for preparing a compound as defined in claim 1, which process involves reacting an acid halide of formula (IIa) or (IIb);

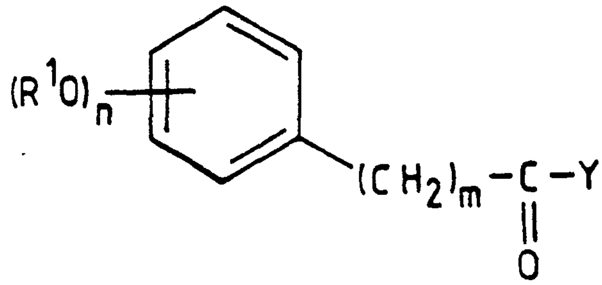

(IIa)

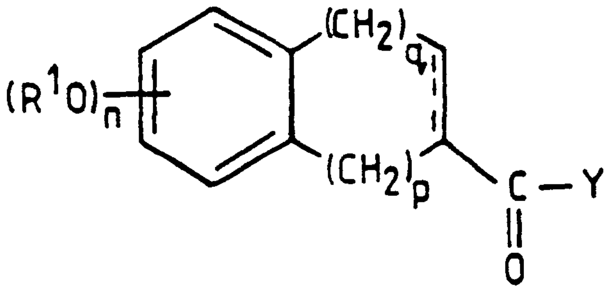

(IIb)

with an amine of formula (III);

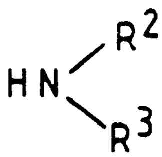

(III)

(where $R^{1'}$ is a hydroxy-protecting group, Y is a halogen atom, and $R^{1'}$, $R^2$, $R^3$, m, n, p and q are as defined in claim 1), optionally salifying the compound, and optionally removing the hydroxy-protecting group represented by $R^{1'}$

13. The use of a compound as defined in claim 1, for the preparation of a pharmaceutical composition by admixture with a pharmaceutically acceptable carrier.